# EUROPEAN PATENT APPLICATION

(11) **EP 4 697 002 A1**
(43) Date of publication of application: **18.02.2026**
(21) Application number: 24937303.6
(22) Date of filing: 16.12.2024
(51) Int. Cl.: G01N 1/10, B01D 53/34, B01D 53/40, B01J 19/00, C08F 2/38, C12M 1/26, G01N 1/14, G01N 1/28

(54) **IN-SITU SAMPLING AND MOLECULAR WEIGHT MEASUREMENT SYSTEM OF BATCH REACTOR**

(30) Priority: 14.06.2024 KR 20240077787
(71) Applicant: LG Chem, Ltd., Seoul 07336 (KR)
(72) Inventor: The designation of the inventor has not yet been filed
(74) Representative: Goddar, Heinz J.
(86) International application number: PCT/KR2024/020399
(87) International publication number: WO 2025/258771

(57) **Abstract**

An in-situ sampling and molecular weight detection system for batch reactor is disclosed. The system includes: a batch reactor including an inlet through which a reactant solution is selectively supplied, and an outlet through which a product formed by a reaction of the reactant solution is selectively discharged; a sampling line connected to the reactor through which the reactant solution in the reaction selectively flows; and a plurality of sampling valves mounted on the sampling line and configured to selectively open and close the sampling line, in which the plurality of sampling valves may be configured to be opened and closed sequentially, starting from the sampling valve relatively close to the batch reactor to the sampling valve relatively far from the batch reactor.

## Description

### [Technical Field]

### CROSS-REFERENCE TO RELATED APPLICATIONS

This application claims priority to and the benefit of Korean Patent Application No. 10-2024-0077787 filed in the Korean Intellectual Property Office on June 14, 2024, the entire contents of which are incorporated herein by reference.

The present disclosure relates to an in-situ molecular weight detection system for a batch reactor, and more particularly, to an in-situ sampling and molecular weight detection system for a batch reactor capable of sampling a sample in real time while maintaining an internal pressure in a high-pressure batch reactor and measuring a molecular weight, etc., of the sampled sample in real time.

### [Background Art]

A batch reactor is a device that performs a chemical reaction. A chemical reaction using a batch reactor generally proceeds through the following process.

First, a reactant is put in the reactor, and uniformly mixed using a mixer. Thereafter, reaction conditions are controlled through a temperature control device and a pressure control device, and an appropriate reaction time is set while the reaction is in progress. Through this process, a desired reaction may be efficiently performed under desired conditions.

In order to set an appropriate reaction time, it is necessary to check the progress of the reaction in real time, but it was difficult to sample internal materials during the reaction in a conventional batch reactor. In particular, it was very difficult to sample internal materials while maintaining the internal pressure for the reaction in the high-pressure batch reactor.

In general, in the case of a conventional batch reactor, when the reaction was completed, the pressure was completely discharged and the internal materials were analyzed. Therefore, it was difficult to directly check the progress of the reaction. Even if it was possible to sample the material inside the reactor during the reaction, it was difficult to analyze a conversion rate, selectivity, or molecular weight information in real time because the sampling time and drying time for analysis were long.

The above information disclosed in this Background Art is only for enhancement of understanding of the background of the invention and therefore it may contain information that does not form the prior art that is already known in this country to a person of ordinary skill in the art.

### [Disclosure]

### [Technical Problem]

The present disclosure attempts to provide to an in-situ sampling and molecular weight detection system for a batch reactor capable of sampling a sample in real time while maintaining an internal pressure in a high-pressure batch reactor and measuring a molecular weight, etc., of the sampled sample in real time.

### [Technical Solution]

According to an exemplary embodiment of the present disclosure, an in-situ sampling and molecular weight detection system for a batch reactor includes: a batch reactor including an inlet through which a reactant solution is selectively supplied, and an outlet through which a product made by a reaction of the reactant solution is selectively discharged; a sampling line connected to the reactor through which the reactant solution in the reaction selectively flows; and a plurality of sampling valves mounted on the sampling line and configured to selectively open and close the sampling line, in which the plurality of sampling valves may be configured to be opened and closed sequentially, from the sampling valve relatively close to the batch reactor to the sampling valve relatively far from the batch reactor.

The in-situ sampling and molecular weight detection system may further include a melt flow index (MFI) meter disposed on the sampling line downstream of the plurality of sampling valves and configured to measure an MFI of the reactant solution flowing through the sampling line.

The plurality of sampling valves may include: a first sampling valve disposed relatively close to the batch reactor; and a second sampling valve disposed relatively close to the MFI meter, in which the first sampling valve may be configured to open and close a portion of the sampling line in which the first sampling valve is positioned, and the second sampling valve may be configured to open and close a portion of the sampling line in which the second sampling valve is positioned, and the second sampling valve may be configured to be opened and closed after the first sampling valve is opened and closed during sampling of the reactant solution.

The in-situ sampling and molecular weight detection system may further include an inert gas supply source connected to the sampling line between the first and second sampling valves through an inert gas line and configured to supply an inert gas to the sampling line.

The inert gas line may be connected to the sampling line between the first and second sampling valves through a cleaning line.

The in-situ sampling and molecular weight detection system may further include a steam supply source connected to the sampling line between the first and second sampling valves through a steam line and configured to supply steam or a cleaning solution to the sampling line.

The steam line may be connected to the sampling line between the first and second sampling valves through a cleaning line.

The in-situ sampling and molecular weight detection system may further include a check valve mounted on the cleaning line to prevent the reactant solution in the sampling line from flowing back into the cleaning line.

The in-situ sampling and molecular weight detection system may further include a heat tracing configured to keep the sampling line warm to a set temperature or higher.

The MFI meter may have a heating function to keep the solution in the MFI meter warm to the set temperature or higher.

The in-situ sampling and molecular weight detection system may further include a pressure sensor mounted on the sampling line upstream of the MFI meter and configured to measure pressure within the sampling line upstream of the MFI meter.

The inert gas line or the steam line may be connected to the sampling line between the first and second sampling valves through a cleaning line, and the system may include: an inert gas valve disposed on the inert gas line and configured to open and close the inert gas line; a steam valve disposed on the steam line and configured to open and close the steam line; and a controller configured to be connected to the pressure sensor to control the first and second sampling valves, the inert gas valve, and/or the steam valve based on the pressure within the sampling line upstream of the MFI meter.

The controller may be connected to the MFI meter and configured to receive the measured MFI and calculate a molecular weight of the reactant solution using the MFI.

### [Advantageous Effects]

According to the present disclosure, the separate sampling line is provided at a lower portion of the batch reactor, the plurality of valves are installed in the sampling line, and when the plurality of valves are sequentially opened to sample a material inside the reactor, the pressure inside the reactor can be prevented from decreasing.

In addition, a melt flow index (MFI) meter is installed in the sampling line so that the molecular weight, etc., may be measured in situ and in real time simultaneously with sampling the material inside the reactor.

In addition, the heat tracing can be installed in the sampling line so that the sampled material may be prevented from solidifying.

In addition, the steam line and the inert gas line may be connected so that the maintenance such as the cleaning of the sampling line is easy.

Other effects that may be obtained or are predicted by an exemplary embodiment of the present disclosure will be explicitly or implicitly described in a detailed description of the present disclosure. That is, various effects that are predicted according to an exemplary embodiment of the present disclosure will be described in the following detailed description.

### [Description of the Drawings]

The exemplary embodiments of the present specification may be better understood with reference to the following description in conjunction with the accompanying drawings, in which like reference numerals refer to identical or functionally similar elements.
FIG. 1 is a schematic diagram of an in-situ sampling and molecular weight detection system for a batch reactor according to an exemplary embodiment of the present disclosure.
FIG. 2 is an enlarged view of part A of FIG. 1.

It should be understood that the drawings referenced above are not necessarily drawn to scale, and present rather simplified representations of various preferred features illustrating the basic principles of the present disclosure. For example, specific design features of the present disclosure, including specific dimensions, direction, position, and shape, will be determined in part by specific intended applications and use environments.

### [Mode for Invention]

The terminology used herein is for the purpose of describing particular embodiments only, and is not intended to limit the present invention. As used herein, singular forms are intended to also include plural forms, unless the context clearly dictates otherwise. The terms "comprises" and/or "comprising," specify the cited features, integers, steps, operations, elements, and/or the presence of components when used herein, but it will also be understood that these terms do not exclude the presence or addition of one or more of other features, integers, steps, operations, elements, components, and/or groups thereof. As used herein, the term "and/or" includes any one or all combinations of the associated listed items.

Additionally, it is understood that one or more of the methods below or aspects thereof may be executed by at least one or more controllers. The term "controller" may refer to a hardware device including a memory and a processor. The memory is configured to store program instructions, and the processor is specifically programmed to execute the program instructions to perform one or more processes described in more detail below. The controller may control operations of units, modules, parts, devices, or the like, as described herein. It is also understood that methods below may be executed by an apparatus including a controller in conjunction with one or more other components, as will be appreciated by those skilled in the art.

In addition, the controller of the present disclosure may be implemented as a non-transitory computer-readable recording medium containing executable program instructions executed by a processor. Examples of computer-readable recording media include, but are not limited to, a ROM, a RAM, a compact disc (CD) ROM, magnetic tapes, floppy disks, flash drives, smart cards, and optical data storage devices. The computer-readable recording medium may also be distributed throughout a computer network so that the program instructions may be stored and executed in a distributed manner, such as a telematics server or a controller area network (CAN).

According to an exemplary embodiment of the present disclosure, an in-situ sampling and molecular weight detection system for a batch reactor is disclosed. The system includes a batch reactor including an inlet through which a reactant solution is selectively supplied, and an outlet through which a product formed by a reaction of the reactant solution is selectively discharged; a sampling line connected to the reactor through which the reactant solution in the reaction selectively flows; and a plurality of sampling valves mounted on the sampling line and configured to selectively open and close the sampling line. The plurality of sampling valves are configured such that the sampling valves relatively close to the batch reactor are opened and closed, and then the sampling valves relatively far from the batch reactor are opened and closed. Accordingly, the amount of reactant solution flowing out through the sampling line during sampling is limited, and the pressure fluctuation inside the batch reactor may be small.

The system further includes a melt flow index (MFI) meter configured to be disposed on a sampling line downstream of the plurality of sampling valves to measure an MFI of the reactant solution flowing through the sampling line. Accordingly, the molecular weight of the reactant solution, etc., may be measured in situ and in real time simultaneously with the sampling of the reactant solution.

The system may further include a heat tracing configured to keep the sampling line warm to a set temperature or higher. Accordingly, the temperature of the reactant solution within the sampling line may be prevented from decreasing, thereby preventing the reactant solution from solidifying within the sampling line.

The plurality of sampling valves may include at least first and second sampling valves. The first sampling valve is relatively close to the batch reactor, and the second sampling valve is relatively close to the MFI meter. The system further includes an inert gas line connected to the sampling line between the first and second sampling valves and connected to an inert gas supply source. By supplying an inert gas to the sampling line upstream of the MFI meter through the inert gas line, the pressure of the sampling line may be maintained constant. In addition, in order to control the supply of the inert gas, the system further includes a pressure sensor configured to measure the pressure of the sampling line in the sampling line upstream of the MFI meter. In addition, the system further includes a steam line connected to the sampling line between the first and second sampling valves and connected to a steam supply source. By supplying the steam to the sampling line upstream of the MFI meter through the steam line, the sampling line may be cleaned.

Hereinafter, an exemplary embodiment of the present disclosure will be described in detail with reference to the attached drawings.

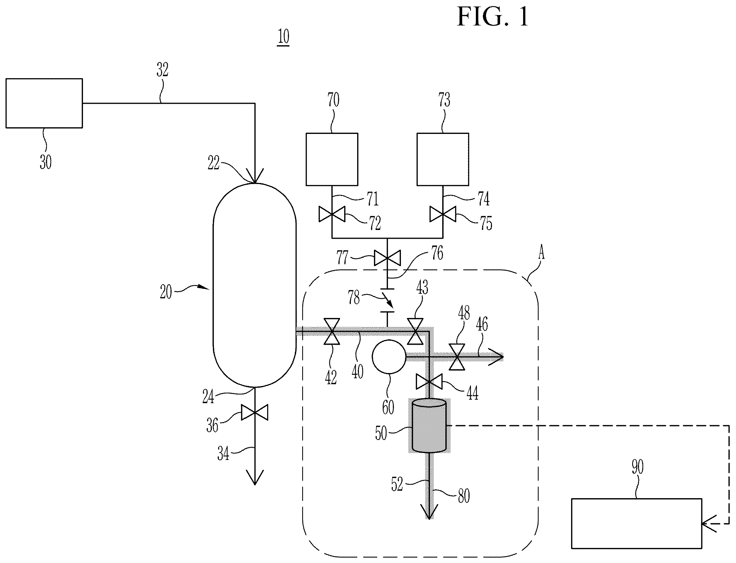

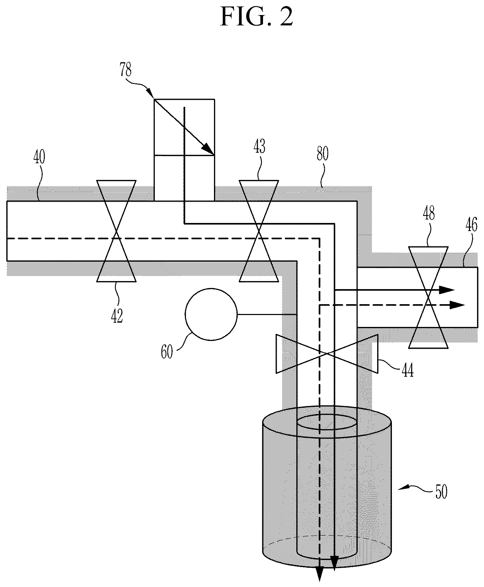

FIG. 1 is a configuration diagram of an in-situ sampling and molecular weight detection system for a batch reactor according to an exemplary embodiment of the present disclosure, and FIG. 2 is an enlarged view of part A of FIG. 1.

As illustrated in FIGS. 1 and 2, an in-situ sampling and molecular weight detection system 10 for a batch reactor according to an exemplary embodiment of the present disclosure includes a batch reactor 20, a reactant supply source 30, a sampling line 40, and a melt flow index (MFI) meter 50.

The batch reactor 20 is a place where a reaction of reactants occurs. An inlet 22 is provided at an upper portion of the batch reactor 20, and a reactant solution is selectively supplied to the batch reactor 20 through the inlet 22. An outlet 24 is provided at a lower portion of the batch reactor 20, and a product produced by the reaction of the reactant solution is discharged from the batch reactor 20 through the outlet 24. The batch reactor 20 may be equipped with a stirring device for stirring the reactant solution, a pressure control device for controlling pressure inside the reactor, and a temperature control device (e.g., a heater, etc.) for controlling temperature inside the reactor.

The reactant supply source 30 stores the reactant and is connected to the inlet 22 of the batch reactor 20 through the supply line 32. The reactant stored in the reactant supply source 30 may be supplied to the batch reactor 20 through the supply line 32 and the inlet 22. For example, when the product is a polyamide strand, the reactant may be adipic acid (ADA), pentamethylene diamine (PMDA), water, etc. The batch reactor may further include a plurality of supply lines 32 including a plurality of reactant supply sources 30, each supply line 32 connecting each reactant supply source 30 to the inlet 22 of the batch reactor 20, and a plurality of supply valves including each supply valve (not illustrated) mounted on each supply line 32 to open and close the corresponding supply line 32.

The outlet 24 of the batch reactor 20 is connected to a drain line 34, and the drain line 34 is equipped with a drain valve 36. When the reaction of the reactant is completed, the drain valve 36 opens the drain line 34 to discharge the product and an unreacted material to the outside of the batch reactor 20, particularly to a water tank, etc., through the drain line 34.

The sampling line 40 is connected to the lower portion of the batch reactor 20, and the reactant solution inside the batch reactor 20 may be selectively sampled through the sampling line 40. The sampling line 40 is equipped with a plurality of sampling valves 42, 43, and 44, and each sampling valve 42, 43, and 44 is positioned at a different position on the sampling line 40 and is configured to open and close the sampling line 40. Here, three sampling valves are provided as an example, but the number of sampling valves is not limited to three. However, for the convenience of description, a case in which three sampling valves 42, 43, and 44 are provided is described as an example.

The first sampling valve 42 is positioned relatively close to the batch reactor 20, the third sampling valve 44 is positioned relatively close to the MFI meter 50, and the second sampling valve 43 is positioned between the first sampling valve 42 and the third sampling valve 44. The first, second, and third sampling valves 42, 43, and 44 are configured so that after the first sampling valve 42 relatively close to the batch reactor 20 is opened and closed, the second sampling valve 43 is opened and closed, and then the third sampling valve 44 is opened and closed. When the first sampling valve 42 is opened and closed, the reactant solution corresponding to the distance between the first and second sampling valves 42 and 43 is sampled, when the second sampling valve 43 is opened and closed, the sampled reactant solution flows into the third sampling valve 44, and when the third sampling valve 44 is opened and closed, the sampled reactant solution flows into the MFI meter 50 (see the dotted line in FIG. 2, where the dotted line in FIG. 2 indicates the flow of the reactant solution). Accordingly, the amount of reactant solution sampled during sampling is limited, and thus the pressure fluctuation inside the batch reactor 20 may be small.

A vent line 46 is connected to a downstream portion of the sampling line 40, and a vent valve 48 configured to open and close the vent line 46 is mounted on the vent line 46. For example, the vent line 46 may be connected to the sampling line 40 between the second sampling valve 43 and the third sampling valve 44, but is not limited thereto. The vent line 46 may be opened by the vent valve 48 when the sampling line 40 is cleaned, so that the molten material remaining in the sampling line 40 may be discharged through the vent line 46. In addition, when the pressure in the sampling line 40 is too high, the vent valve 48 may open the vent line 46 to discharge a portion of the reactant solution in the vent line 46 to the outside, thereby lowering the pressure in the sampling line 40 (see the dotted line in FIG. 2).

The MFI meter 50 is mounted on the sampling line 40 downstream of the third sampling valve 44. The MFI meter 50 is connected to the sampling line 40, and the reactant solution flowing through the sampling line 40 flows into the MFI meter 50. In addition, the MFI meter 50 is connected to an MFI drain line 52, and the reactant solution flowing into the MFI meter 50 and passing through the MFI meter 50 may be discharged to the outside through the MFI drain line 52 (see the dotted line in FIG. 2). The MFI meter 50 is configured to measure the MFI of the reactant solution passing through the MFI meter 50. The MFI is related to the time it takes for the reactant solution to pass through the MFI meter 50. When the MFI is large, the flowability of the melt is good, which means that the molecular weight of the melt is small. In contrast, when the MFI is small, the flowability of the melt is poor, which means that the molecular weight of the melt is large. Therefore, the molecular weight of the melt may be indirectly measured by measuring the MFI of the melt. In this way, according to an exemplary embodiment of the present disclosure, when sampling the reactant solution using the plurality of sampling valves 42, 43, and 44, the MFI of the sampled reactant solution may also be measured in-situ through the MFI meter 50. Accordingly, the molecular weight of the reactant solution, etc., may be measured in situ and in real time simultaneously with the sampling of the reactant solution.

In order to measure the molecular weight, etc., of the reactant solution in-situ using the MFI meter 50, the reactant in the sampling line 40 should be present in a molten state. For this purpose, the system 10 further includes a heat tracing 80 configured to supply heat to at least the sampling line 40, preferably the sampling line 40, the vent line 46, and the MFI drain line 52 to keep the temperature to a set temperature or higher. For example, in the case of a polyamide polymerization reaction, the set temperature may be 260°C, but is not limited thereto. The set temperature may be set according to the reaction occurring in the batch reactor 20.

The heat tracing 80 is not limited to its type as long as it is a device capable of keeping the solution within the sampling line 40, the vent line 46, and the MFI drain line 52 to the set temperature or higher. For example, the heat tracing 80 may be a double jacket that surrounds the sampling line 40, the vent line 46, and the MFI drain line 52 and allows the heat to flow therein, or may be a heat transfer band wound around the sampling line 40, the vent line 46, and the MFI drain line 52. The heat tracing 80 keeps the solution within the sampling line 40, the vent line 46, and the MFI drain line 52 to the set temperature or higher to prevent the solution from solidifying within the lines 40, 46, and 52 and blocking the lines 40, 46, and 52. In addition, the MFI meter 50 also has a heat retention function to keep the solution therein to the set temperature or higher.

The system 10 may further include a steam supply source 70 for supplying steam to the sampling line 40 and an inert gas supply source 73 for supplying an inert gas to the sampling line 40.

The steam supply source 70 is connected to the sampling line 40 through a steam line 71, and a steam valve 72 is mounted on the steam line 71 to open and close the steam line 71. The steam line 71 is connected to the sampling line 40 downstream of the first sampling valve 42, preferably to the sampling line 40 between the first sampling valve 42 and the second sampling valve 43, such that the steam supply source 70 supplies steam to the sampling line 40 between the first sampling valve 42 and the second sampling valve 43 through the steam line 71 to clean the sampling line 40, the MFI meter 50, the MFI drain line 52, and/or the vent line 46 with steam (see solid lines in FIG. 2, where solid lines represent the flow of steam or inert gas). Alternatively, the steam source 70 may be used to inject a cleaning solution (e.g., triethylene glycol, etc.) into the sampling line 40 via the steam line 71 instead of or in addition to steam to clean the sampling line 40, the MFI meter 50, the MFI drain line 52, and/or the vent line 46.

The inert gas source 73 is connected to the sampling line 40 via an inert gas line 74, and the inert gas line 74 is equipped with an inert gas valve 75 to open and close the inert gas line 74. The inert gas line 74 is connected to the sampling line 40 downstream of the first sampling valve 42, preferably, to the sampling line 40 between the first sampling valve 42 and the second sampling valve 43, such that the inert gas supply source 73 may supply an inert gas to the sampling line 40 between the first sampling valve 42 and the second sampling valve 43 through the inert gas line 74. For example, when the pressure in the sampling line 40 is low and the reactant solution in the sampling line 40 does not flow smoothly into the MFI meter 50, the inert gas supply source 73 supplies an inert gas to the sampling line 40 through the inert gas line 74 to maintain the pressure of the sampling line 40 constant, thereby allowing the reactant solution in the sampling line 40 to flow smoothly into the MFI meter 50. In addition, the inert gas supplied to the sampling line 40 through the inert gas line 74 may clean the sampling line 40, the MFI meter 50, the MFI drain line 52, and/or the vent line 46 (see the solid line in FIG. 2). Here, the inert gas may be, but is not limited to, nitrogen.

The steam line 71 and the inert gas line 74 are joined by a cleaning line 76 and connected to the sampling line 40 through the cleaning line 76. The cleaning line 76 is equipped with a cleaning valve 77 to open and close the cleaning line 76. For example, when the pressure of the sampling line 40 is low or the reactant solution does not flow smoothly into the MFI meter 50, the cleaning valve 77 opens the cleaning line 76 so that the inert gas and/or steam is supplied to the sampling line 40. In this case, depending on the required operation, the steam valve 72 may open the steam line 71 and/or the inert gas valve 75 may open the inert gas line 74.

The cleaning line 76 may further be equipped with a check valve 78. The check valve 78 is mounted on the cleaning line 76 adjacent to the sampling line 40 to prevent the reactant solution in the sampling line 40 from flowing back into the cleaning line 76.

In this specification, the steam line 71 and the inert gas line 74 are joined to the cleaning line 76 and indirectly connected to the sampling line 40 through the cleaning line 76, but each of the steam line 71 and the inert gas line 74 may be directly connected to the sampling line 40 without going through the cleaning line 76. In this case, the steam line 71 and the inert gas line 74 may each be equipped with a check valve 78.

The system 10 further includes a pressure sensor 60 and a controller 90.

The pressure sensor 60 is mounted on the sampling line 40 upstream of the MFI meter 50 to measure the pressure in the sampling line 40 upstream of the MFI meter 50 and transmit a signal thereof to the controller 90.

The controller 90 is connected to the pressure sensor 60 to receive a signal about the pressure in the sampling line 40 upstream of the MFI meter 50. The controller 90 may be connected to valves in the system 10, such as the drain valve 36, the first, second, and third sampling valves 42, 43, and 44, the vent valve 48, the steam valve 72, the inert gas valve 75, and/or the cleaning valve 77, to control the operation of the valves. In one example, the controller 90 may control the drain valve 36 to open the drain line 34 in response to completion of the target reaction within the batch reactor 20. In another example, the controller 90 may control the sequential opening and closing of the first, second, and third sampling valves 42, 43, and 44 in response to receiving a request for sampling and molecular weight measurement of the reactant solution. In another example, the controller 90 may control the inert gas valve 75 to open the inert gas line 74, the cleaning valve 77 to open the cleaning line 76, and the inert gas supply source 73 to supply the inert gas to the sampling line 40 in response to determining that the pressure within the sampling line 40 measured by the pressure sensor 60 is lower than a lower pressure limit. In another example, the controller 90 may control the vent valve 48 to open the vent line 46 in response to the determination that the pressure within the sampling line 40 measured by the pressure sensor 60 is lower than an upper pressure limit, thereby reducing the pressure within the sampling line 40. In another example, the controller 90 may, in response to the determination that cleaning of the sampling line 40 is necessary (e.g., completion of sampling and molecular weight measurement of the reactant solution or when the flow of the reactant solution within the sampling line 40 is not smooth, etc.), control the inert gas valve 75 to open the inert gas line 74, and/or control the steam valve 72 to open the steam line 71, control the cleaning valve 77 to open the cleaning line 76, control the second and third sampling valves 43 and 44 to open the sampling line 40 downstream of the first sampling valve 42, control the vent valve 48 to open the vent line 46, control the inert gas supply source 73 to supply inert gas to the sampling line 40, or control the steam supply source 70 to inject steam or a cleaning solution into the sampling line 40.

In addition, the controller 90 is connected to the MFI meter 50 and may calculate the molecular weight corresponding to the MFI measured by the MFI meter 50.

### (Examples and Comparative Examples)

According to Examples 1 to 3, adipic acid (ADA), pentamethylene diamine (PMDA), water, etc., were supplied to the batch reactor 20 according to the general polyamide manufacturing method, the reactant solution was stirred, and the temperature and pressure inside the batch reactor 20 were controlled so that the polymerization reaction was conducted, and the reactant solution was sampled through the sampling line 40 while the **MFI** of the reactant solution was measured through the **MFI** meter 50.

According to Comparative Examples 1, 3, and 5, the polyamide was manufactured according to the polyamide manufacturing method, the manufactured polyamide was melted, and the **MFI** of the polyamide solution was measured using the conventional **MFI** meter.

According to Comparative Examples 2, 4, and 6, polyamide was manufactured according to the polyamide manufacturing method, the manufactured polyamide was melted, and the relative viscosity (based on sulfuric acid) of the polyamide solution was measured using a relative viscosity meter.

The experimental results of the examples and comparative examples are listed in Table 1.

**[Table 1]**

| | Measurement time | Sample drying time | Sampling time | Total sample analysis time | Analysis result | Error (%) |
|---|---|---|---|---|---|---|
| Example 1 | 5 minutes | 0 minutes | 2 minutes | 7 minutes | 80(g/10min @ 270°C) | 12 |
| Comparative Example 1 | 10 minutes | 720 minutes | 180 minutes | 910 minutes | 80(g/10min @ 270°C) | 10 |
| Comparative Example 2 | 240 minutes | 720 minutes | 180 minutes | 1140 minutes | 2.76 (based on sulfuric acid) | 1.6 |
| Example 2 | 5 minutes | 0 minutes | 2 minutes | 7 minutes | 105(g/10min @ 270°C) | 15 |
| Comparative Example 3 | 8 minutes | 720 minutes | 180 minutes | 908 minutes | 110.4(g/10min @ 270°C) | 13.6 |
| Comparative Example 4 | 240 minutes | 720 minutes | 180 minutes | 1140 minutes | 2.50 (based on sulfuric acid) | 1.6 |
| Example 3 | 5 minutes | 0 minutes | 2 minutes | 7 minutes | 50(g/10min @ 270°C) | 10 |
| Comparative Example 5 | 12 minutes | 720 minutes | 180 minutes | 912 minutes | 51.7(g/10min @ 270°C) | 9 |
| Comparative Example 6 | 240 minutes | 720 minutes | 180 minutes | 1140 minutes | 2.94 (based on sulfuric acid) | 1.6 |

Referring to Table 1, in the case of Examples 1 to 3, since the sampling is performed in situ during the reaction of the reactant solution, the sampling time is very short at 2 minutes (sequential opening and closing time of the first, second, and third sampling valves 42, 43, and 44), but in the case of the comparative examples, since the completed polyamide is used after the polymerization reaction of the polyamide is completed, the sampling time is the polymerization reaction time of the polyamide. Accordingly, the sampling time of the comparative examples is very long at 180 minutes.

In the case of the examples, since sampling and MFI measurement are performed simultaneously during the reaction of the reactant solution, there is no sample drying time, but in the case of the comparative examples, the moisture content of the polyamide solution should be lowered to measure the MFI or relative viscosity, so at least 10 hours or more are required.

The total sample time is the sum of the measurement time, the sample drying time, and the sampling time. In the case of the Examples, the total sample time is very short at about 7 minutes, but in the case of Comparative Example 1, Comparative Example 3, and Comparative Example 5, the total sample analysis time is very long at 900 minutes or more, and in the case of Comparative Example 2, Comparative Example 4, and Comparative Example 6, the total sample analysis time is the longest at about 1140 minutes. In contrast, the error is the highest at about 15% for the Examples, somewhat high at about 14% for Comparative Example 1, Comparative Example 3, and Comparative Example 5, and very low at 1.6% for Comparative Example 2, Comparative Example 4, and Comparative Example 6. Since the present disclosure is to check the progress of the reaction in real time by sampling and measuring molecular weight in situ, the Examples are sufficient to achieve the purpose of the present disclosure when the total sample time, error, etc., are taken into consideration, but it is difficult to achieve the purpose of the present disclosure in the Comparative Examples because the sampling and measuring molecular weight may not be performed in situ.

While this invention has been described in connection with what is presently considered to be practical embodiments, it is to be understood that the invention is not limited to the disclosed embodiments. On the contrary, it is intended to cover various modifications and equivalent arrangements included within the spirit and scope of the appended claims.

## Claims

1. An in-situ sampling and molecular weight detection system for a batch reactor, comprising:
a batch reactor including an inlet through which a reactant solution is selectively supplied, and an outlet through which a product made by a reaction of the reactant solution is selectively discharged;
a sampling line connected to the reactor through which the reactant solution in the reaction selectively flows; and
a plurality of sampling valves mounted on the sampling line and configured to selectively open and close the sampling line,
wherein the plurality of sampling valves is configured to be opened and closed sequentially, from the sampling valve relatively close to the batch reactor to the sampling valve relatively far from the batch reactor.

2. The in-situ sampling and molecular weight detection system of claim 1, further comprising:
a melt flow index (MFI) meter disposed on the sampling line downstream of the plurality of sampling valves and configured to measure an MFI of the reactant solution flowing through the sampling line.

3. The in-situ sampling and molecular weight detection system of claim 2, wherein:
the plurality of sampling valves includes:
a first sampling valve disposed relatively close to the batch reactor; and
a second sampling valve disposed relatively close to the MFI meter,
wherein the first sampling valve is configured to open and close a portion of the sampling line in which the first sampling valve is positioned, and the second sampling valve is configured to open and close a portion of the sampling line in which the second sampling valve is positioned, and
the second sampling valve is configured to be opened and closed after the first sampling valve is opened and closed during sampling of the reactant solution.

4. The in-situ sampling and molecular weight detection system of claim 3, further comprising:
an inert gas supply source connected to the sampling line between the first and second sampling valves through an inert gas line and configured to supply an inert gas to the sampling line.

5. The in-situ sampling and molecular weight detection system of claim 4, wherein:
the inert gas line is connected to the sampling line between the first and second sampling valves through a cleaning line.

6. The in-situ sampling and molecular weight detection system of claim 3, further comprising:
a steam supply source connected to the sampling line between the first and second sampling valves through a steam line and configured to supply steam or a cleaning solution to the sampling line.

7. The in-situ sampling and molecular weight detection system of claim 6, wherein:
the steam line is connected to the sampling line between the first and second sampling valves through a cleaning line.

8. The in-situ sampling and molecular weight detection system of claim 5 or 7, further comprising:
a check valve mounted on the cleaning line to prevent the reactant solution in the sampling line from flowing back into the cleaning line.

9. The in-situ sampling and molecular weight detection system of claim 2, further comprising:
a heat tracing configured to keep the sampling line warm to a set temperature or higher.

10. The in-situ sampling and molecular weight detection system of claim 9, wherein:
the MFI meter has a heating function to keep the solution in the MFI meter warm to the set temperature or higher.

11. The in-situ sampling and molecular weight detection system of claim 4 or 6, further comprising:
a pressure sensor mounted on the sampling line upstream of the MFI meter and configured to measure pressure within the sampling line upstream of the MFI meter.

12. The in-situ sampling and molecular weight detection system of claim 11, wherein:
the inert gas line or the steam line is connected to the sampling line between the first and second sampling valves through a cleaning line, and
the system includes:
an inert gas valve disposed on the inert gas line and configured to open and close the inert gas line;
a steam valve disposed on the steam line and configured to open and close the steam line; and
a controller connected to the pressure sensor and configured to control the first and second sampling valves, the inert gas valve, and/or the steam valve based on the pressure within the sampling line upstream of the MFI meter.

13. The in-situ sampling and molecular weight detection system of claim 12, wherein:
the controller is connected to the MFI meter to receive the measured MFI and configured to calculate a molecular weight of the reactant solution using the MFI.
